# EUROPEAN PATENT SPECIFICATION

(11) **EP 2 016 946 B1**
(45) Date of publication and mention of the grant of the patent: **27.02.2013**
(21) Application number: 07014046.2
(22) Date of filing: 18.07.2007
(51) Int. Cl.: A61K 31/722, A61K 33/00, A61K 31/365, A61P 3/04, A61P 3/06, A61P 39/02

(54) **Chitosan silicon dioxide coprecipitate composition for use as a therapeutically active agent**
Chitosan-Siliziumdioxid-Co-Präzipitat-Zusammensetzung zur Verwendung als therapeutischer Wirkstoff
Composition de coprécipité de chitosane et de dioxyde de silicium pour une utilisation en tant qu'agent thérapeutique

(43) Date of publication of application: 21.01.2009
(73) Proprietor: The Jordanian Pharmaceutical Manufacturing Co., 11710 Naor (JO)
(72) Inventor: Badwan, Adnan Dr., 11185 Amman (JO); Al-Remawi, Mayyas Dr., 13713 Russiefa (JO); Taha, Hashem Dr., 11821 Amman (JO)
(74) Representative: Winkler, Andreas Fritz Ernst

(56) References cited:
- EP-A- 1 516 882
- WO-A-00/09123
- WO-A-00/24785
- WO-A-99/36075
- WO-A-03/097077
- WO-A-2007/025715
- WO-A-2007/132479
- WO-A1-2007/074326
- WO-A2-2005/117844
- DE-A1-102004 014 483
- FR-A- 2 874 925
- US-A1- 2003 022 574
- DATABASE WPI 11 September 1987 (1987-09-11), Derwent Publications Ltd., London, GB; Class 874,page 2, AN 1987-295464 XP002462553 OCHI SHIGEO: "Obesity preventive agent" & JP 62 207220 A (OCHI SHIGEO) 11 September 1987 (1987-09-11)
- DATABASE WPI 10 May 2006 (2006-05-10), Derwent Publications Ltd., London, GB; Class 063,page 6, AN 2006-349372 XP002462554 LISETSKAYA T.A. ET AL.: "Enterosorbent for heavy metal excretion" & RU 2 275 916 C (MEGRABYAN K.A.) 10 May 2006 (2006-05-10)

## Description

The present invention relates in general to the field of medicine. More precisely, the present invention relates to a chitosan silicon dioxide coprecipitate composition having a high fat binding ability for the use as a therapeutically active agent. In particular, the present invention concerns a chitosan silicon dioxide coprecipitate composition for the use in the manufacture of a medicament for the treatment of overweight, diseases related thereto as specified in the claims, and poisonings with fat soluble toxic materials.

### BACKGROUND OF THE INVENTION

Obesity is a condition in which the natural energy reserve, stored in the fatty tissue of humans and other mammals, is increased to a point where it is associated with certain health conditions or increased mortality. In its simplest conception, obesity occurs when the lifetime energy intake exceeds the lifetime energy expenditure by more than it does for individuals of "normal weight". In case the food energy intake exceeds the energy expenditure, fat cells (and to a lesser extent muscle and liver cells) throughout the body take in the energy and store it as fat.

However, there are also many factors that have been suggested to contribute to the development of obesity. Such factors are for instance genetic factors, genetic disorders (e.g. Prader-Willi syndrome), an underlying illness (e.g. hypothyroidism), eating disorders (e.g. binge eating disorder), certain medications (e.g. atypical antipsychotics, some fertility medication), sedentary lifestyle, a high glycemic diet (i.e. a diet that consists of meals that give high postprandial blood sugar), weight cycling, caused by repeated attempts to lose weight by dieting, stressful mentality, insufficient sleep, smoking cessation, and in addition, not being physically active.

Obesity is considered as a chronic disease and is one of the most serious contributors to increased morbidity and mortality. Obese and overweight human subjects suffer from increased joint problems, such as high blood pressure, high blood cholesterol, and triglyceride levels (combined hyperlipidemia). Increased weight is also associated with cardiovascular diseases, diabetes mellitus type 2, stroke, sleep apnea, and osteoarthritis. Alone in the United States approximately 97 million people are considered to be clinically obese and overweight.

Usually obesity requires long-term treatment in order to promote and to sustain weight loss. The mainstay of treatment for obesity is an energy-limited diet and increased exercise. Beyond this, there are also some chemical drugs available which are suitable for the treatment of obesity.

There are appetite suppressants, such as phendimetrazine, diethylpropion, phentermine and sibutramine which are the most commonly prescribed appetite-suppressants in the U.S and which are approved by the Food and Drug Administration (FDA), however, these drugs have a high potential for abuse and dependence.

Further, there are lipase inhibitors, such as orlistat, which reduce the ability of the body to absorb dietary fat by blocking the enzyme lipase. Lipase is responsible for breaking down dietary fat in order to become absorbable. However, the side effects of orlistat include cramping, intestinal discomfort, passing gas, diarrhea, and leakage of oily stool. These side effects are generally mild and temporary, but may be worsened by eating fat-rich food. In addition, since orlistat also reduces the absorption of some vitamins, patients should take a multivitamin tablet at least 2 hours before or after taking orlistat in order to avoid any deficiency symptom.

In addition, there are further medications used for the treatment of obesity such as some antidepressants, antiseizures, and antidiabetes. However, these drugs are not approved by the FDA with respect to their use for the treatment of obesity. Their use in order to loose weight is only an "off-label"-use, i.e. a term refers to the practice of prescribing medication for periods of time or for conditions not FDA-approved.

The other option to combat obesity is the replacement of fat-rich diets with low-fat diets. However, the presence of fats in many food sources greatly limits the food sources, which can be used in a low-fat diet. Additionally, fat contributes to the flavor, appearance and physical characteristics of many foodstuffs. As such, the acceptability of low-fat diets and the maintenance of such diets are difficult.

Therefore, beside the above-mentioned means for the treatment of obesity and diseases related thereto the approach of reducing the absorption of the fat associated with the diet seems to be a promising tool. Furthermore, the approach of reducing the absorption of fat can be also used for the treatment of poisonings. Toxic materials such as pesticides and insecticides are fat soluble. Thus, compounds having a fat binding ability are also suitable to prevent the absorption of such fat soluble toxic materials.

There are some patents with respect to the use of compositions which are suitable to bind fatty materials. US patent 7,048,917 discloses fat-binding polymers with respect to a method for the treatment of obesity. Further, US patent 7,049,345 discloses fat-binding polymers comprising dialkanolamine, dialkanolammonium, aminoalkylpolyol, and ammoniumalkylpolyol pendant groups for subjects in need of fat removal from the gastrointestinal tract, particularly subjects suffering from steatorrhea (oily stool) and/or experiencing side effects from lipase inhibitors. In addition, US patent 7,144,865 discloses compositions for the treatment of obesity, wherein the compositions comprise: (1) zinc salt, (2) cyclo-Hispro and/or (3) arachidonic acid, and (4) at least one pharmaceutically acceptable excipient.

There is another important and naturally occurring compound known to be suitable to bind fatty materials: chitosan either administered in solid or liquid dosage forms. EP1226810 relates to a composition for entrapping oil and/or fat comprising a solvent, chitosan and at least one polymer selected from sodium alginate, xanthan and mixtures thereof.

Chitosan is a natural substance which is available in nature and in addition, which is inexpensive, non-toxic, biodegradable, biocompatible in comparison to other polymers. Further, chitosan is considered to be non-digestible by a human if administered orally.

WO-A-99/36075 discloses an oil sorbent agent which is delivered to the large or small intestine to absorb unwanted dietary fat or to reduce the side-effects of lipase-inhibitors. The oil sorbent agent is calcium silicate or microcrystalline chitosan.

JP-A-62 207 220 teaches that silica gel is used as an adsorbent capable of absorbing nutriments, digested and decomposed in intracorporeal digestive organs. The medicament is a preventive for obesity.

WO200702571 discloses a method of coprecipitation of a chitosan silicon dioxide composition and a directly compressible material of a chitosan silica coprecipitate as an excipient. This method of coprecipitation of chitosan with silica was found to increase the particle size, to improve the flow properties and to increase the chitosan compressibility properties and the disintegration ability.

As outlined above, the drugs for the treatment of obesity are associated with undesirable side effects. Therefore, it would be an advantage to provide a new drug effective to combat obesity which has none or at least low side effects and which is at the same time easy to handle in the manufacture of a medicament. Thus, it is an object of the present invention to provide a novel drug or composition suitable to bind fatty materials and thus suitable to be used as a therapeutically active agent in the manufacture of a medicament for the treatment of overweight, obesity, diseases related thereto, and, in addition, suitable to be used as an antidote of fat soluble toxic materials.

### SUMMARY OF THE INVENTION

The object of the present invention, is solved by a chitosan silicon dioxide coprecipitate composition for use in the treatment of overweight, obesity, hypertriglyceridemia, steatorrhea, side effects of lipase inhibitors, and poisonings with fat soluble toxic materials.

### DESCRIPTION OF THE PREFERRED EMBODIMENTS OF THE INVENTION

Unless defined otherwise, all technical and scientific terms used herein have the same meanings as commonly understood by one of ordinary skill in the art.

As outlined above, there is a need in the prior art to provide a new drug or composition suitable to bind fatty materials and thus suitable to be used for the treatment of obesity, and diseases related thereto. Such drug or composition, as mentioned above, is in parallel suitable to be used as an antidote of fat soluble toxic materials, such as pesticides or insecticides.

It was found, surprisingly, that there is a synergistic fat binding ability for a chitosan silicon dioxide coprecipitate composition compared to the fat binding ability of either of chitosan or silicon dioxide when used separately. As outlined above, the fat binding ability of chitosan alone was already known, however, a chitosan silicon dioxide coprecipitate composition was only reported to be suitable as an excipient. The synergistic fat binding ability of a chitosan silicon dioxide coprecipitate composition was totally unexpected.

Therefore, in a first aspect the present invention provides a chitosan silicon dioxide coprecipitate composition in the treatment of overweight, obesity, hypertriglyceridemia, steaborrhea, side effects of lipase inhibitors and poisonings with fat soluble toxic materials.

In a preferred embodiment the chitosan silicon dioxide composition according to the present invention comprises chitosan material having a degree of deacetylation (DDA) in the range between 0 to 100%, preferably between 50 to 95%.

In another preferred embodiment the chitosan silicon dioxide composition according to the present invention comprises chitosan material having an average molecular weight ranging from 1.000 to 10.000.000 g/mol, preferably from 2.000 to 1.000.000 g/mol.

The efficacy of the used chitosan materials depends on the pH of the preparation media. Preferably the pH is acidic, typically in the range of 4 to 6.8. However, basic ranges of pH, typically above 8, may be preferable for the preparation of chitosan primary amine base.

In one embodiment the chitosan silicon dioxide composition according to the present invention comprises chitosan material being chitosan or its pharmaceutically acceptable salts.

In another embodiment the chitosan silicon dioxide composition according to the present invention comprises silicon dioxide being present in crystalline or amorphous form or as a modified form or salt of silicon dioxide.

In one preferred embodiment the chitosan silicon dioxide composition according to the present invention comprises silicon dioxide, which is present in the form of colloidal silicon dioxide. Preferably, the specific surface area of the colloidal silicon dioxide typically comprise from 5 to 2000 m²/g, more preferably from 5-500 m²/g, most preferably from 50 to 400 m²/g.

In one embodiment the chitosan silicon dioxide coprecipitate composition according to the present invention further comprises one or more fillers, lubricants, fat absorbent materials, and pH buffering agents. These optional agents may facilitate and/or improve the physical properties or efficiency of the fat binding ability of the chitosan silicon dioxide composition.

The chitosan silicon dioxide coprecipitate composition according to the present invention can be prepared by any method of preparation known in the art. The most preferable methodology for preparation is described in WO2007025715. Surprisingly, it was found that the chitosan silicon dioxide coprecipitate composition, which was so far only known as an excipient, has a super binding ability to fatty materials. Therefore, in order to be used as an inert excipient this composition should be taken two hours before the ingestion of fat-rich meals.

In addition, the chitosan silicon dioxide coprecipitate composition according to the present invention is directly compressible and thus easy to handle in the manufacture of a medicament, such as tablet dosage forms, which in addition, are considered to be the most convenient form for both manufacturers and patients.

In one embodiment of chitosan silicon dioxide coprecipitate composition according to the present invention the weight percentage of chitosan is in the range between 10% up to 90%, preferable between 30-60%.

Also disclosed is a pharmaceutical composition comprising, as an active agent, the chitosan silicon dioxide coprecipitate composition .

The pharmaceutical composition may further comprise at least one lipase inhibitor. Preferably, the at least one lipase inhibitor is selected from the group consisting of orlistat, lipstatin and tetrahydrostatin.

The pharmaceutical composition may further comprise one or more of fillers, lubricants, fat absorbent materials, pH buffering agents and any excipient suitable in the manufacture of a pharmaceutical composition with respect to the dosage form and in addition, known by the person skilled in the art. Preferably, the pharmaceutical composition is an oral dosage form, such as a powder, granules, solution, suspension, syrup, and lozenges. More preferably, the pharmaceutical composition is a disintegrating tablet dosage form as known by the person skilled in the art.

Also disclosed is the use of a chitosan silicon dioxide coprecipitate according to the present invention in the manufacture of a medicament for the treatment, prevention or alleviation of overweight, obesity, hypertriglyceridemia, steatorrhea, side effects of lipase inhibitors, and poisonings with fat soluble toxic materials.

The main side effect of lipase inhibitor is oily stool or steatorrhea, some minor side effects including cramps, discomfort, passing gas and diarrhea. The chitosan silicon dioxide coprecipitate composition according to the present invention is suitable to minimize the side effects of lipase inhibitors, in particular, to minimize oily stool or steatorrhea caused by lipase inhibitors. Therefore, co-administering of the chitosan silicon dioxide coprecipitate composition according to the present invention and lipase inhibitors increases the compliance of the lipase inhibitors.

Patients being administered with lipase inhibitors are typically being treated for Type II diabetes, streatorrhea, and hypertriglyceridemia. The chitosan silicon dioxide coprecipitate composition according to the present invention is suitable to be administered to such subjects in order to reduce the side effects of lipase inhibitors. In addition, the chitosan silicon dioxide coprecipitate composition according to the present invention is also suitable to be used alone for the treatment of obese subjects.

Administration of the chitosan silicon dioxide coprecipitate composition according to the present invention facilitates the removal of fat from the body prior to digestion with minimal side effects and low toxicity. The composition according to the present invention is composed of two materials with synergistic actions in fat binding i.e. chitosan material and silicon dioxide.

Since the chitosan silicon dioxide coprecipitate composition according to the present invention facilitates the removal of fat from the body prior to digestion, the absorption of fat soluble toxic materials will be prevented by the rapid ingestion of such composition. Thus, the synergistic fat binding ability of the chitosan silicon dioxide coprecipitate composition according to the present invention allows its use as an antidote of fat soluble toxic materials, and thus its use in the manufacture of a medicament for the treatment of poisonings with fat soluble toxic materials, such as pesticides and insecticides.

Administering the chitosan silicon dioxide coprecipitate composition according to the present invention to a human reduces the absorption of dietary fat.

In one embodiment of the present invention, the chitosan silicon dioxide coprecipitate composition according to the present invention is administered oral. Preferably, the chitosan silicon dioxide composition or pharmaceutical composition is administered by a oral dosage form known in the art, such as powder, granules, solution, suspension, syrup, and lozenges.

In a preferred embodiment of the present invention, the chitosan silicon dioxide coprecipitate composition according to the present invention is administered prior to the ingestion of a fat-rich diet. The fatty materials and the bile salt are the proposed targets for binding to the chitosan silicon dioxide composition. The binding to the chitosan silicon dioxide coprecipitate composition will prevent their absorption via the gastrointestinal system. The administration of the chitosan silicon dioxide coprecipitate composition according to the present invention will facilitate the removal of fat from the body prior to digestion, with negligible side effects.

In one embodiment of the present invention , the chitosan silicon dioxide coprecipitate composition according to the present invention is administered after the exposure to fat soluble toxic materials. Since the fat binding ability of the composition according to the present invention facilitates the removal of fat from the body prior to digestion, the absorption of fat soluble toxic materials will be prevented by a rapid ingestion of such composition.

In another embodiment the treatment further comprises administering a therapeutically effective amount of a lipase inhibitor. Preferably, the lipase inhibitor is selected from the group consisting of orlistat, lipstatin and tetrahydrostatin.

### BRIEF DESCRIPTION OF THE DRAWING

Figure 1 shows a photograph of samples after centrifugation upon adding 3 volume milk precipitation of a full cream milk preparation at a concentration of 10% w/w in phosphate buffer pH 6.8 to 2 volume acid soluble portion of different fat absorption materials at a concentration of 1% w/w.
Figure 2 shows the turbidity of the supernatant as an indication of the fat absorption for different fat binding materials.
Figure 3 shows the relative capacity of the fat binding ability based on turbidity measures after centrifugation of 3 volume of full cream milk preparation of a concentration of 10% w/w in phosphate buffer pH 6.8 upon mixing with 2 volumes of the acid soluble portion of different fat absorption materials at a concentration of 1% w/w.
Figure 4 shows the final pH-values of the supernatant of different fat absorption materials

### EXAMPLES

### Example 1

### Fat binding procedure

1 gram of a polymer or composition required for evaluation as a fat binding polymer was dissolved in 100 ml 0.1 M HCl in a 250 ml E. flask and stirred until the sample was completely dissolved, otherwise, the samples were centrifuged at 2000 rpm for 1min and the supernatants were taken. In another 250 ml E. flask 10 gram of full cream milk powder (NIDEO-Nestle, Netherlands) was added to 100 ml phosphate buffer at pH 6.8 and stirred until a homogeneous dispersion was obtained. A constant volume ratio of the acidic solution containing the sample was added to the buffered milk dispersion 2:3 v/v and was gently mixed in a 10 ml screw capped tubes. In another experiment, the pH of the final solution was measured (the pH was in the range of 5 to 6). The tubes were immediately centrifuged at 2000 rpm for 1 min. The supernatant turbidity was taken as a measure for milk precipitation. A blank sample of milk was treated similarly without the addition of fat binding samples. Another set of controls that did not contain milk was treated similarly using a phosphate buffer at pH 6.8. These tubes were used as a turbidity background for the real samples and they were referred as controls. In addition, the supernatant turbidity (100 -%T) was determined. % T means the percent transmittance at 600 nm.

### Explanation to the above mentioned procedure

First, dissolving the fat binding polymer in 0.1 M HCl.: the fat binding composition should be first dissolved in the acidic condition of the stomach. 0.1 M HCl medium was used to resemble the acidic condition of the stomach. In most cases the fat binding polymer is soluble in acidic medium.

Second, selection of milk preparation: the model meal used herein can be easily dispersed in a suspension form and it is rich in fat, i.e. full-cream milk powder (NIDEO-Nestle, Netherlands), each 100 gram dried milk contains 28.8% fat.

Third, suspending milk in phosphate buffer pH 6.8: in normal situations the fat binding composition is given prior to ingestion of the fat-rich meal. In this acidic condition the polymer will dissolve. Then the fat-rich meal will be ingested at acidic pH. The binding between chitosan and fatty materials is minimal in the stomach due to the acidic pH. However, the pH is increased and reaches about 5 to 6 in the duodenum stage and this pH is considered the optimum pH for chitosan and fatty material binding. We resembled the duodenum stage by dissolving the milk powder in phosphate buffer pH 6.8. The addition of 2 volumes of the sample in acidic 0.1 M HCl to the 3 volumes of the buffered milk always results in a final pH between 5 and 6, which is the preferable pH for the chitosan silicon dioxide composition in the duodenal stage.

Fourth, centrifugation and turbidity measurement: the binding of the soluble fat binding materials to milk fatty colloids results in an increase of the size of the colloids. Applied mild centrifugation will separate bounded milk large particles. The unbound milk colloids with small size will not precipitate easily. This makes blank milk preparation treated without the addition of a fat binding polymer to appear turbid even after centrifugation in comparison with samples containing the fat binding polymers.

The degree of turbidity of the supernatant can be taken as a measure for fat binding capability for each fat binding polymer as an in-vitro test.

### Example 2

### Supernatant clarity as indication of fat binding

The same procedure for comparison of fat binding ability described previously in example 1 was applied for the following fat binding polymers or compositions A) chitosan, B) Minus Fat ®, C) Liponet ®, D) Chitosan silica coprecipitate (1:1 w/w), E) Silica, and F) Milk control.

B and C are commercial products used for fat binding containing chitosan and other additives.

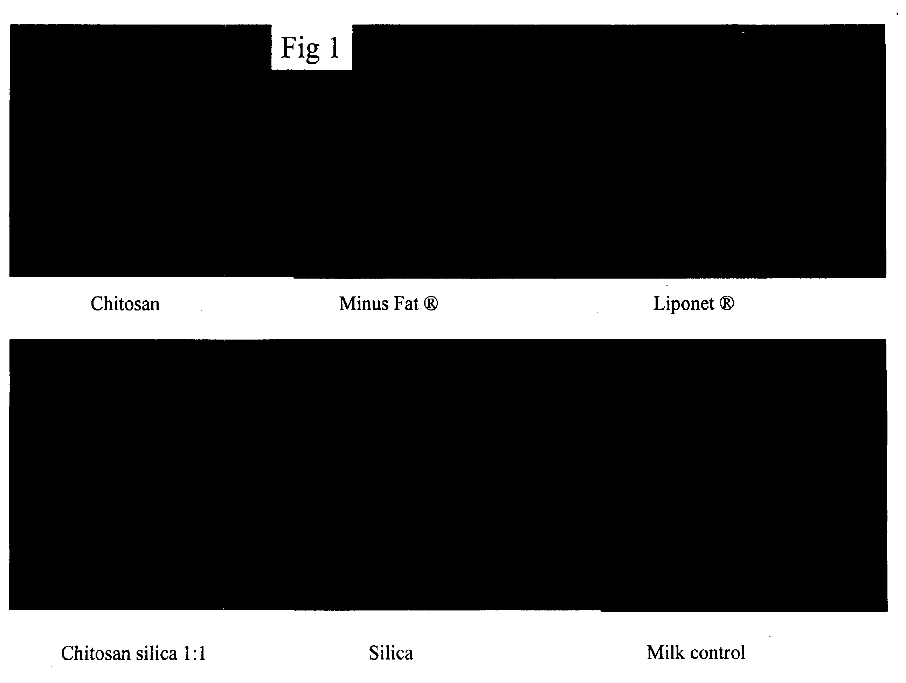

Figure 1 shows the samples after centrifugation. The supernatant clarity can be easily distinguished for both Liponet ® and Chitosan silica coprecipitate (1:1 w/w). This means those two compositions will have the superiority for fat binding over other preparations. Another important note is that chitosan or silica as a single preparation does not result in a high fat binding while the combination of chitosan and silicon dioxide resulted in a pronounced synergistic ability for fat binding.

### Example 3

### Turbidity determination

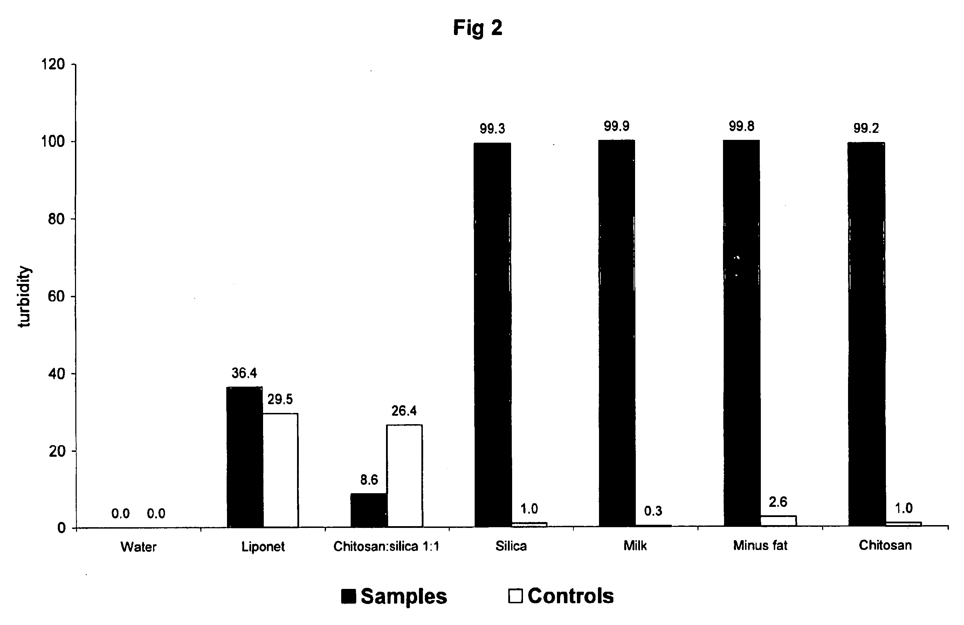

Turbidity measurements were taken for samples and their controls as described previously in examples 1 and 2 as shown in Figure 2. The samples with lowest turbidity indicates the maximum fat uptake ability. Chitosan silica 1:1 w/w attained the maximum fat uptake ability followed by Liponet®. This ensures the superiority of the novel combination of chitosan and silica in fat binding.

### Example 4

### Milk binding capacity calculation according to example 3

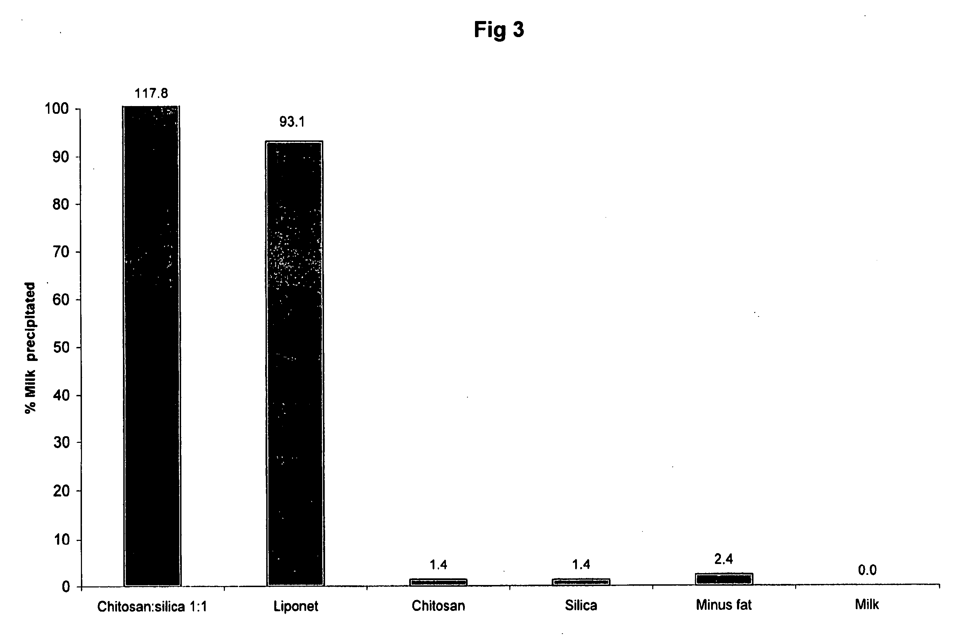

The turbidity measurements of samples were subtracted from their controls to obtain the effective turbidity. The milk control turbidity was subtracted from the effective turbidity of the samples and then divided again on the milk control turbidity to obtain a value that indicates the fat binding capacity as shown in Figure 3. The maximum fat binding capacity is for chitosan:silica 1:1. This clearly indicates its superior fat uptake over other preparations.

### Example 5

### Final pH values

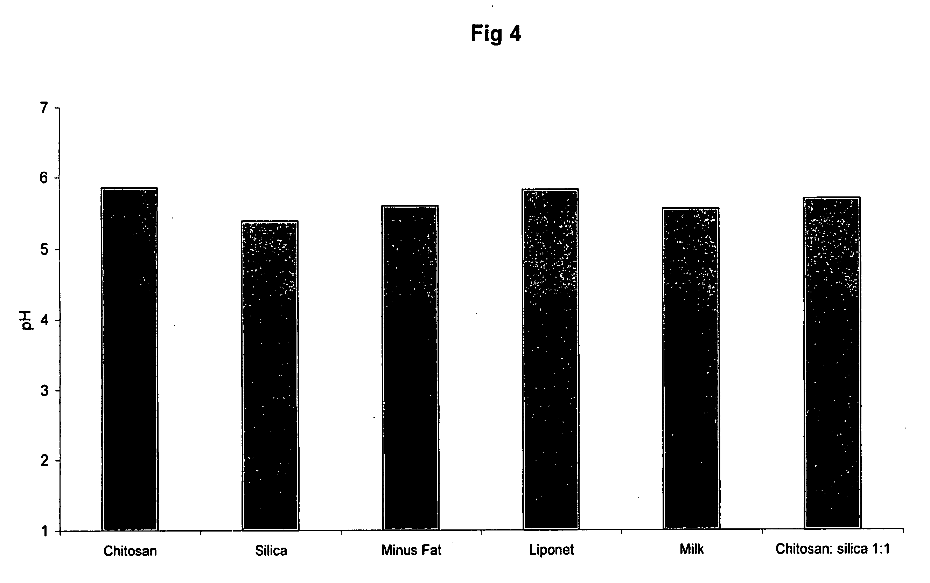

For all samples the final pH value was determined after mixing the buffered milk with the acidic solution containing the fat binding materials as described above in example 1. There were no big differences in the pH that might result in the binding at different pH value, as shown in Figure 4.

This suggests that the only changing condition is the fat binding polymer and its ability to capture the fatty material at nearly similar pH values.

As a conclusion a super fat binding ability was observed for chitosan:silica 1:1 w/w in comparison to other commercial products. In addition, a synergistic fat binding was observed by comparing chitosan:silica 1:1 w/w with that of chitosan and silica as an individual fat uptake materials.

## Claims

1. Chitosan silicon dioxide coprecipitate composition for use in the treatment of overweight, obesity, hypertriglyceridemia, steatorrhea, side effects of lipase inhibitors and poisonings with fat soluble toxic materials.

2. Composition for use according to claim 1, wherein said composition comprises chitosan material being chitosan or a pharmaceutically acceptable salt thereof.

3. Composition for use according to any of claims 1 or 2, wherein said composition comprises chitosan material having a degree of deacetylation in the range between 0 to 100%, preferably between 50 to 95%.

4. Composition for use according to any of claims 1 to 3, wherein said composition comprises chitosan material having an average molecular weight ranging from 1.000 to 10.000.000 g/mol, preferably from 2.000 to 1.000.000 g/mol.

5. Composition for use according to any of claims 1 to 4, wherein said composition comprises silicon dioxide being present in crystalline or amorphous form or as a modified form or salt of silicon dioxide.

6. Composition for use according to claim 5, wherein the silicon dioxide is present in the form of colloidal silicon dioxide.

7. Composition for use according to any of claims 1 to 6, wherein said composition further comprises one or more of fillers, lubricants, fat absorbent materials, and pH buffering agents.

## Patentansprüche

1. Chitosan-Siliciumdioxid-Co-Präzipitat-Zusammensetzung zur Verwendung bei der Behandlung von Übergewicht, Fettleibigkeit, Hypertriglyceridämie, Steatorrhoe, Nebenwirkungen von Lipase-Inhibitoren und Vergiftungen mit fettlöslichen toxischen Materialien.

2. Zusammensetzung zur Verwendung nach Anspruch 1, wobei die Zusammensetzung Chitosan-Material umfasst, das Chitosan oder ein pharmazeutisch verträgliches Salz davon ist.

3. Zusammensetzung zur Verwendung nach einem der Ansprüche 1 oder 2, wobei die Zusammensetzung Chitosan-Material mit einem Deacetylierungsgrad im Bereich zwischen 0 bis 100 %, vorzugsweise zwischen 50 bis 95 % umfasst.

4. Zusammensetzung zur Verwendung nach einem der Ansprüche 1 bis 3, wobei die Zusammensetzung Chitosan-Material mit einem durchschnittlichen Molekulargewicht im Bereich von 1.000 bis 10.000.000 g/mol, vorzugsweise von 2.000 bis 1.000.000 g/mol umfasst.

5. Zusammensetzung zur Verwendung nach einem der Ansprüche 1 bis 4, wobei die Zusammensetzung Siliciumdioxid umfasst, das in kristalliner oder amorpher Form oder als eine modifizierte Form oder ein Salz von Siliciumdioxid vorliegt.

6. Zusammensetzung zur Verwendung nach Anspruch 5, wobei das Siliciumdioxid in der Form von kolloidalem Siliciumdioxid vorliegt.

7. Zusammensetzung zur Verwendung nach einem der Ansprüche 1 bis 6, wobei die Zusammensetzung weiter einen oder mehrere Füllstoffe, Gleitmittel, Fettabsorbtionsmaterialien und pH-Puffermittel umfasst.

## Revendications

1. Composition à base d'un coprécipité de chitosane et de dioxyde de silicium à utiliser dans le traitement de l'excès de poids, de l'obésité, de l'hypertriglycéridémie, de la stéatorrhée, des effets secondaires des inhibiteurs de la lipase et des empoisonnements avec des matières toxiques liposolubles.

2. Composition à utiliser selon la revendication 1, dans laquelle ladite composition comprend un matériau de chitosane ou un sel pharmaceutiquement acceptable correspondant.

3. Composition à utiliser selon l'une des revendications 1 ou 2, dans laquelle ladite composition comprend un matériau de chitosane ayant un degré de déacétylation compris dans la plage allant de 0 à 100%, de préférence de 50 à 95%.

4. Composition à utiliser selon l'une des revendications 1 à 3, dans laquelle ladite composition comprend un matériau de chitosane ayant un poids moléculaire moyen allant de 1000 à 10 000 000g/mol, de préférence de 2000 à 1 000 0OOg/mo

5. Composition à utiliser selon l'une des revendications 1 à 4, dans laquelle ladite composition comprend un dioxyde de silicium qui est présent sous forme cristalline ou amorphe ou sous forme modifiée ou d'un sel de dioxyde de silicium.

6. Composition à utiliser selon la revendication 5, dans laquelle le dioxyde de silicium est présent sous forme de dioxyde de silicium colloïdal.

7. Composition à utiliser selon l'une des revendications 1 à 6, dans laquelle ladite composition comprend en outre un ou plusieurs éléments parmi les charges, les lubrifiants, les matériaux absorbant les graisses, et les agents de tamponnage de pH.
